(19) Europäisches Patentamt
European Patent Office
Office européen des brevets

(11) Veröffentlichungsnummer: **0 023 016**
**B1**

(12) # EUROPÄISCHE PATENTSCHRIFT

(45) Veröffentlichungstag der Patentschrift: **16.06.82**

(51) Int. Cl.³: **C 09 B 7/10,** C 07 D 333/74, C 07 D 333/78, C 07 D 333/64

(21) Anmeldenummer: **80104105.4**

(22) Anmeldetag: **15.07.80**

(54) **Verfahren zur Herstellung asymmetrischer Thioindigoverbindungen sowie Thionaphthenone an sich.**

(30) Priorität: **18.07.79 CH 6682/79**

(43) Veröffentlichungstag der Anmeldung: **28.01.81 Patentblatt 81/4**

(45) Bekanntmachung des Hinweises auf die Patenterteilung: **16.06.82 Patentblatt 82/24**

(84) Benannte Vertragsstaaten: **CH DE FR GB LI**

(56) Entgegenhaltungen:
**DE-A-2 804 842**
**FR-A-390 484**
**FR-A-823 196**
**GB-A-2 022 606**
**US-A-2 539 737**

(73) Patentinhaber: **CIBA-GEIGY AG, Patentabteilung Postfach, CH-4002 Basel (CH)**

(72) Erfinder: **Bühler, Niklaus, Dr., Gartenweg 30, CH-4310 Rheinfelden (CH)**
Erfinder: **Bosshard, Hans, Hohe Winde-Strasse 23, CH-4059 Basel (CH)**

(74) Vertreter: **Zumstein sen., Fritz, Dr. et al, Bräuhausstrasse 4, D-8000 München 2 (DE)**

**EP 0 023 016 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

# Verfahren zur Herstellung asymmetrischer Thioindigoverbindungen sowie Thionaphthenone an sich

Die vorliegende Erfindung betrifft ein neues Verfahren zur Herstellung von asymmetrischen Thioindigoverbindungen. Die erfindungsgemäss herstellbaren Thioindigoverbindungen finden unter anderem Anwendung als Küpen-, Dispersions-, Spinnmasse- und Pigmentfarbstoffe. Asymmetrische Thioindigoverbindungen zeichnen sich gegenüber entsprechenden symmetrischen Verbindungen im allgemeinen durch niedrigere Schmelzpunkte, eine bessere Löslichkeit in organischen Lösungsmitteln, einen höheren Molekularabsorptionskoeffizienten, grössere Farbstärke und Brillanz aus und eignen sich besonders zum Färben von Polyestermaterialien (vgl. z.B. DE-OS 2 401 981).

Asymmetrische Thioindigoverbindungen können nach verschiedenen Verfahren hergestellt werden, z.B. durch oxidative Kondensation von zwei verschieden substituierten Thianaphthenonen-3 oder deren Carbonsäuren, durch oxidative Kondensation von Thianaphthenonen-3 mit Thianaphthenchinonen oder 2,2-Dibrom-thianaphthenonen-3. Dabei entstehen jedoch Gemische von symmetrischen und asymmetrischen Thioindigoverbindungen, die sich nur schwer trennen lassen. Gemäss einem weiteren Verfahren werden Thiosalicylsäuren mit 1,2-Dichloräthylen umgesetzt, worauf man das Reaktionsprodukt mit Chlorsulfonsäure zur Thioindigoverbindung dehydriert [vgl. z.B. Rood's Chemistry of Carbon Compounds, Bd. IV, Part B, 359-360 (1977)]. Dieses letztere Verfahren hat wegen der beschränkten Zugänglichkeit und der nicht ungefährlichen Herstellung von substituierten Thiosalicylsäuren (Umsetzung von diazotierten substituierten Anthranilsäuren mit Thioverbindungen) nur geringe wirtschaftliche Bedeutung erlangt. Die wichtigste Methode zur Herstellung von asymmetrischen Thioindigoverbindungen ist das sogenannte Anilverfahren, d.h. die oxidative Kondensation von Thianaphthenonen-3 mit einem Anil eines Thianaphthenons, z.B. 2-(4'-dimethylaminophenylimino)-thianaphthenon-3. Bei der Herstellung des Anils aus den Thianaphthenonen durch Umsetzung mit geeigneten Nitrosoverbindungen fallen als Nebenprodukt auch symmetrische Thioindigoverbindungen an. Ausserdem sind Umsetzungen mit Nitrosoverbindungen aus ökologischen Gründen unerwünscht (vgl. z.B. Houben-Weyl, 7/4, S. 39).

Die Thianaphthenone-3 ihrerseits werden durch Cyclisieren von Phenylthioglykolsäuren in Gegenwart von Schwefelsäure oder Chlorsulfonsäure oder durch Überführen der Phenylthioglykolsäuren in die entsprechenden Säurechloride und Friedel-Crafts-Ringschluss der Säurechloride hergestellt. Dabei müssen zur Verhinderung einer teilweisen vorzeitigen Oxidation des entstandenen Thianaphthenons und damit der Bildung erheblicher Anteile an symmetrischen Thioindigoverbindungen spezielle Massnahmen ergriffen werden (vgl. z.B. FIAT, 1313, II, 297). Diese vorbekannten Verfahren eignen sich nicht oder nur schlecht für eine grosstechnische Anwendung.

Es wurde nun ein einfaches und wirtschaftliches Verfahren gefunden, nach dem sich asymmetrische Thioindigoverbindungen in hoher Reinheit, mit guten Ausbeuten und auch unter ökologisch günstigeren Bedingungen herstellen lassen.

Gegenstand der Erfindung ist somit ein Verfahren zur Herstellung von Verbindungen der Formel I

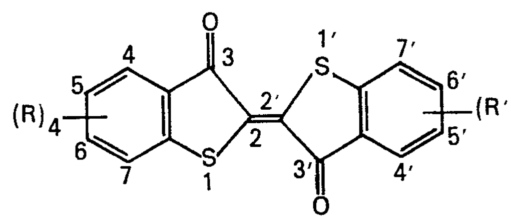 (I),

worin

zwei R bzw. R' Wasserstoff oder Alkyl mit 1-12 C-Atomen,

ein R bzw. R' Wasserstoff, ein Halogenatom oder Alkyl mit 1-12 C-Atomen und

ein R bzw. R' Wasserstoff, ein Halogenatom, Alkyl, Alkoxy, Alkylthio, Alkoxyalkyl, Alkanoyloxyalkyl oder Alkylcarbamoyl mit je 1-12 C-Atomen in den Alkyl- und Alkoxyteilen und 2-13 C-Atomen in den Alkanoylteilen oder zwei benachbarte Substituenten R bzw. R' zusammen Trimethylen oder Tetramethylen und die restlichen R Wasserstoff darstellen, mit der Massgabe, dass mindestens ein R ungleich einem Substituenten R' ist, indem man eine Verbindung der Formel II

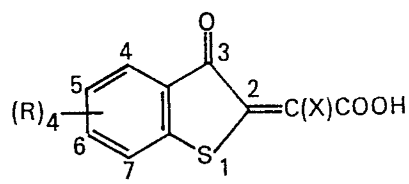 (II)

mit einer Verbindung der Formel III

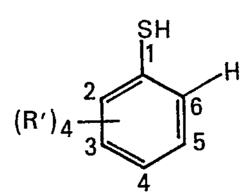 (III)

zu einer Verbindung der Formel IV

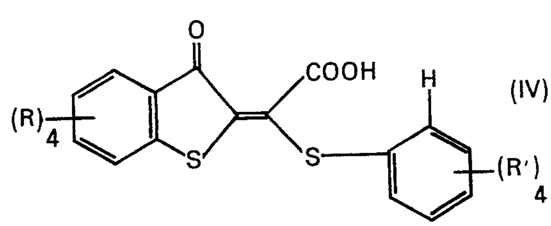 (IV)

umsetzt, die Verbindung der Formel IV entweder direkt durch Wasserabspaltung, bevorzugt in Gegenwart eines wasserabspaltenden Mittels, zu einer Verbindung der Formel I cyclisiert oder die Verbindung der Formel IV in das entsprechende Säurechlorid überführt und das Säurechlorid in Gegenwart eines Friedel-Crafts-Katalysators zu einer Verbindung der Formel I cyclisiert, wobei X Chlor, Brom oder Fluor bedeutet und für R bzw. R′ das unter Formel I Angegebene gilt.

Dabei ist überraschend, dass man die Verbindungen der Formel I dadurch aufbauen kann, dass man – entgegen den vorbekannten Verfahren – die zentrale Doppelbindung nicht in der Endstufe der Synthese durch oxidative Kondensation von zwei Thianaphthenonhälften, sondern bereits in der Anfangsstufe einführt und anschliessend die beiden Heterocyclen durch Cyclisierung bildet. Es ist auch überraschend, dass diese Cyclisierung der Verbindungen der Formel IV nicht unter Eliminierung der Doppelbindung zur Bildung einer Spiro-3,3-benzthien-3′-on-yl-2,3-dihydrobenzothiophen-2-carbonsäure führt, da gemäss Literaturangaben (vgl. z.B. G.A. Olah: Friedel Crafts and related Reactions, Bd. III 1, 579–81, New York 1963–65) Benzoylacrylsäuren unter wasserabspaltenden Bedingungen immer an der Doppelbindung reagieren und keine Ringschlüsse unter Beteiligung der Carboxylgruppe ergeben.

Die Verbindungen der Formel I fallen fast ausschliesslich in der trans-Form an. Bei den Zwischenprodukten der Formel IV handelt es sich im allgemeinen um cis/trans-Gemische. Die Zwischenprodukte der Formel IV sind neu und ebenfalls Gegenstand der Erfindung.

Durch R oder R′ dargestellte Alkyl-, Alkoxy-, Alkylthio-, Alkoxyalkyl-, Alkanoyloxyalkyl- oder Alkylcarbamoylgruppen können geradkettig oder verzweigt sein, sind aber bevorzugt geradkettig. Alkyl- und Alkoxygruppen R und R′ sowie Alkyl- und Alkoxyteile in Substituenten R und R′ weisen bevorzugt 1–8 und insbesondere 1–4 C-Atome auf, während Alkanoylteile mit Vorteil 2–9 und vor allem 2–5 C-Atome aufweisen. Als Beispiele von definitionsgemässen Alkyl-, Alkoxy-, Alkylthio-, Alkoxyalkyl-, Alkanoyloxyalkyl- oder Alkylcarbamoylgruppen R oder R′ seien genannt: Methyl, Äthyl, n-Propyl, Isopropyl, n-Butyl, sek-Butyl, n-Pentyl, Neopentyl, n-Hexyl, n-Heptyl, 3-Heptyl, n-Octyl, n-Decyl, n-Dodecyl; Methoxy, Äthoxy, n-Propoxy, n-Butoxy, n-Hexyloxy, n-Octyloxy und n-Dodecyloxy; Methylthio, Äthylthio, n-Propylthio, Isopropylthio, n-Butylthio, n-Pentylthio, n-Hexylthio, n-Heptylthio, n-Decylthio; Methoxymethyl, 2-Methoxyäthyl, 2- oder 3-Methoxypropyl, 3-Äthoxypropyl, 2-Äthoxyäthyl, 2-n-Propoxyäthyl, 2-n-Butoxyäthyl, 2-sek-Butoxyäthyl, 4-Methoxybutyl, 4-Äthoxybutyl, 2-Octyloxyäthyl; Acetyloxymethyl, 2-Acetyloxyäthyl, 2-Propionyloxyäthyl, 2-Butyroxyäthyl; Methylcarbamoyl, Äthylcarbamoyl, n-Propylcarbamoyl, Isobutylcarbamoyl, n-Butylcarbamoyl, n-Hexylcarbamoyl, n-Octylcarbamoyl, n-Nonylcarbamoyl und n-Dodecylcarbamoyl.

Stellt ein R oder R′ eine Alkoxyalkyl- oder Alkanoyloxyalkylgruppe dar, so handelt es sich insbesondere um Gruppen $-(CH_2)_2-O-$Alkyl oder

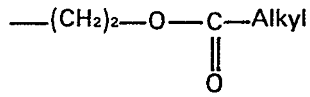

mit je 1–4 C-Atomen in den Alkylteilen.

Als Halogenatome X, R oder R′ kommen z.B. Fluor, Chlor oder Brom in Betracht. X stellt bevorzugt Chlor dar, während als Halogenatome R oder R′ Brom und besonders Chlor bevorzugt sind.

Bevorzugt verwendet man im erfindungsgemässen Verfahren Verbindungen der Formel II und/oder III, worin zwei R bzw. R′ Wasserstoff oder Alkyl mit 1–4 C-Atomen, ein R bzw. R′ Wasserstoff, Chlor, Brom oder Alkyl mit 1–4 C-Atomen und ein R bzw. R′ Wasserstoff, Chlor, Brom, Alkyl, Alkoxy, Alkylthio, $-(CH_2)_2-O-$Alkyl,

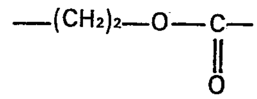

Alkyl oder -NHCO-Alkyl mit je 1–4 C-Atomen in den Alkylteilen und X Chlor darstellen.

Stellen in Formel II oder III zwei benachbarte Substituenten R oder R′ zusammen Trimethylen oder Tetramethylen dar, so haben vorzugsweise die R bzw. R′ in 5,6- bzw. 3,4-Stellung die genannte Bedeutung, und die restlichen R und R′ stellen Wasserstoff dar.

Bevorzugt verwendet man Verbindungen der Formel II und/oder III, worin höchstens drei Substituenten R bzw. R′ ungleich Wasserstoff sind. Sind drei Substituenten R bzw. R′ ungleich Wasserstoff, so können diese in beliebiger Stellung an den entsprechenden Benzolkern gebunden sein.

Gemäss einer weiteren Bevorzugung setzt man Verbindungen der Formel II bzw. III ein, worin zwei R bzw. R′ Wasserstoff bedeuten und die restlichen R bzw. R′ ungleich Wasserstoff sind, mit der Massgabe, dass die R bzw. R′ in 4- und 5-Stellung nicht gleichzeitig ungleich Wasserstoff sind. Besonders bevorzugt sind disubstituierte Verbindungen der Formel II und/oder III, worin entweder die R in 5- und 6-Stellung und die R′ in 3- und 4-Stellung Wasserstoff und die restlichen R bzw. R′ ungleich Wasserstoff sind, oder worin die R in 4- und 7-Stellung bzw. die R′ in 2- und 5-Stellung Wasserstoff und die restlichen R bzw. R′ ungleich Wasserstoff sind.

Unter den monosubstituierten Verbindungen der Formel II und III werden solche bevorzugt, worin das R in 5-, 6- oder 7-Stellung bzw. das R′ in 2-, 3- oder 4-Stellung ungleich Wasserstoff ist.

In den oben erwähnten bevorzugten mono-, di- und trisubstituierten Verbindungen der Formel II und III und den entsprechenden Zwischenprodukten der Formel IV haben von Wasserstoff verschiedene Substituenten R bzw. R′ mit Vorteil die oben angegebene bevorzugte Bedeutung.

Besonders bevorzugt verwendet man Verbindungen der Formel II und III, worin X Chlor, die R in 4- und 7-Stellung Wasserstoff, das R in 5-Stellung Wasserstoff, Chlor, Brom oder Alkyl mit 1-4 C-Atomen, das R in 6-Stellung Waserstoff, Alkyl oder Alkylthio mit je 1-4 C-Atomen oder die R in 5- und 6-Stellung zusammen Trimethylen und die restlichen R Wasserstoff, die R′ in 2- und 5-Stellung Wasserstoff, das R′ in 3-Stellung Wasserstoff oder Alkyl mit 1-4 C-Atomen und das R′ in 4-Stellung Chlor, Brom, -NHCO-Alkyl oder $-(CH_2)_2$-OCO-Alkyl mit je 1-4 C-Atomen in den Alkylteilen oder die R′ in 2- und 5-Stellung Chlor oder Brom und die R′ in 3- und 4-Stellung Wasserstoff bedeuten, sowie Verbindungen der Formel II und III, worin X Chlor, die R in 4- und 7-Stellung Alkyl mit 1-4 C-Atomen, die R′ in 2- und 5-Stellung Wasserstoff, Chlor oder Brom und die restlichen R und R′ Wasserstoff bedeuten.

Die Ausgangsprodukte der Formel III sind bekannt oder können nach üblichen Methoden hergestellt werden. Die Ausgangsprodukte der Formel II sind ebenfalls teilweise bekannt und können nach dem in der DE-A 2 804 842 beschriebenen Verfahren erhalten werden.

Die Umsetzung der Verbindungen der Formel II mit einer Verbindung der Formel III wird mit Vorteil in Gegenwart eines inerten organischen Lösungsmittels bei Temperaturen zwischen etwa 0 und 120°C, besonders etwa 20 und 70°C, vorgenommen. Als inerte organische Lösungsmittel können z.B. verwendet werden: aliphatische Monocarbonsäuren mit 1-4 C-Atomen im Alkylteil und Alkylester von aliphatischen Monocarbonsäuren mit insgesamt 2-6 C-Atomen, wie Essigsäure, Propionsäure, Buttersäure, Ameisen- oder Essigsäuremethyl-, äthyl- und -n-butylester; nieder aliphatische Alkohole und Diole, wie Methanol, Äthanol, n-Propanol, Isopropanol, n-, iso- und tert-Butanol sowie Äthylenglykol; aliphatische Äther, wie Diäthyl- und Di-isopropyläther; Äthylenglykolmono- und -dialkyläther mit je 1-4 C-Atomen in den Alkylteilen, wie Äthylenglykolmonomethyl- und -monoäthyläther, Äthylenglykoldimethyl- und -diäthyläther; aliphatische Ketone, wie Aceton und Methyläthylketon; chlorierte aliphatische Kohlenwasserstoffe, wie Chloroform, 1,2-Dichloräthan, 1,1,2,2-Tetrachloräthan und Tetrachlorkohlenstoff; N,N-Dialkylamide von aliphatischen Monocabonsäuren mit 1-3 C-Atomen im Säureteil, wie N,N-Dimethylformamid, N,N-Dimethylacetamid und N,N-Diäthylacetamid; Dialkylsulfoxide, wie Dimethyl- und Diäthylsulfoxid. Bevorzugtes Lösungsmittel ist wasserfreie Essigsäure.

Die Verbindungen der Formel II und III werden zweckmässig in äquimolaren Mengen eingesetzt; es kann aber auch mit einem etwa 1,1- bis 5-fachen molaren Überschuss in Verbindung der Formel II gearbeitet werden.

Die Zwischenprodukte der Formel IV fallen im allgemeinen in Form von orangeroten bis roten Kristallen an, die auf übliche Weise isoliert und gegebenenfalls gereinigt werden können, z.B. durch Umkristallisation. Eine solche Isolierung und Reinigung ist jedoch nicht unbedingt erforderlich.

Die Cyclisierung der Zwischenprodukte der Formel IV durch Wasserabspaltung wird bevorzugt in Gegenwart eines wasserabspaltenden Mittels vorgenommen. Als wasserabspaltende Mittel können z.B. Polyphosphorsäure, konzentrierte Schwefelsäure, Phosphorpentoxid oder aliphatische Carbonsäureanhydride, wie Essigsäureanhydrid und Propionsäureanhydrid, verwendet werden. Die Cyclisierung in Gegenwart eines wasserabspaltenden Mittels kann mit oder ohne Zusatz eines inerten organischen Lösungsmittels durchgeführt werden, zweckmässig bei Temperaturen zwischen etwa 100 und 300°C, besonders etwa 120 und 270°C. Geeignete inerte organische Lösungsmittel sind z.B. gegebenenfalls chlorierte aliphatische oder aromatische Kohlenwasserstoffe, wie 1,1,2,2-Tetrachloräthan, o-Dichlorbenzol, Trichlorbenzole, Xylole und Nitrobenzol. Die Wasserabspaltung kann aber auch durch blosses Erhitzen mit oder ohne Zusatz eines hochsiedenden organischen Lösungsmittels bei Temperaturen zwischen etwa 100 und 300°C, besonders etwa 150 und 270°C erfolgen. Dafür geeignete Lösungsmittel sind z.B. Trichlorbenzole, Diphenyläther, 2-Chlornaphthalin und Chinolin.

Bevorzugt werden die Verbindungen der Formel IV jedoch zuerst durch Behandlung mit einem Chlorierungsmittel, wie Thionylchlorid, Oxalylchlorid, Phosgen oder Phosphorpentachlorid, in die entsprechenden Säurechloride übergeführt, gegebenenfalls in Gegenwart eines inerten organischen Lösungsmittels, bei Temperaturen zwischen etwa 0 und 100°C, vor allem etwa 0 und 80°C. Als inerte organische Lösungsmittel kommen z.B. in Betracht: N,N-Dialkylamide von aliphatischen Monocarbonsäuren mit 1-3 C-Atomen im Säureteil, wie N,N-Dimethylformamid, N,N-Dimethylacetamid oder N,N-Diäthylacetamid; chlorierte aromatische oder aliphatische Kohlenwasserstoffe, wie 1,2-Dichloräthan, Dichlormethan, Chloroform oder Chlorbenzol. Bevorzugt wird die Chlorierung in Dichloräthan mit Thionylchlorid und unter Zusatz von etwa N,N-Dimethylformamid vorgenommen.

Die Cyclisierung der Säurechloride in Gegenwart eines Friedel-Crafts-Katalysators (Lewis-Säuren) kann in einem inerten organischen Lösungsmittel oder in der Schmelze vorgenommen werden. Als Lewis-Säuren können beispielsweise verwendet werden: Aluminiumtrichlorid, Aluminiumtribromid, Zinkchlorid, Zinntetrachlorid, Bortrifluorid, Eisen(III)chlorid, Titantetrachlorid, Phosphortrichlorid, Phosphoroxychlorid, Antimonpentafluorid und Antimonpentachlorid. Bevorzugt verwendet man Aluminiumtrichlorid. Die Lewis-Säure wird zweckmässig im Überschuss eingesetzt, z.B. in einer etwa 2- bis 10-fachen molaren Menge.

Geeignete organische Lösungsmittel für die

Cyclisierung in Gegenwart eines Friedel-Crafts-Katalysators sind z.B.: chlorierte aliphatische oder aromatische Kohlenwasserstoffe, wie Dichlormethan, 1,2-Dichloräthan, 1,2,3-Trichlorpropan, 1,1,2,2-Tetrachloräthan und o-Dichlorbenzol; n-Pentan und n-Hexan; Nitromethan, Nitrobenzol und Schwefelkohlenstoff.

Die Cyclisierung in der Schmelze wird zweckmässig in Gegenwart von tiefschmelzenden Salzgemischen vorgenommen, z.B. Gemischen von Aluminiumtrichlorid mit anorganischen oder organischen Salzen, wie Ammonium-, Erdalkalimetall- und Alkalimetallhalogenide, beispielsweise Ammonium-, Magnesium-, und Calciumchlorid, insbesondere jedoch Natrium- und Kaliumchlorid, sowie Pyridiniumsalze, z.B. N-Alkylpyridiniumhalogenide.

Bevorzugt sind eutektische Salzgemische, vor allem Gemische aus Aluminiumtrichlorid und Natriumchlorid und/oder Kaliumchlorid. Es können aber an sich beliebige Salzgemische eingesetzt werden, sofern damit eine ausreichende Schmelzpunkterniedrigung erzielt wird.

Vorzugsweise wird die Cyclisierung in Gegenwart von Friedel-Crafts-Katalysatoren jedoch in einem inerten organischen Lösungsmittel, besonders Dichlormethan, 1,2-Dichloräthan oder 1,1,2,2-Tetrachloräthan, oder aber in der Schmelze unter Zusatz von Natrium- und/oder Kaliumchlorid durchgeführt.

Die Cyclisierung der Säurechloride der Verbindungen der Formel IV zu Verbindungen der Formel I wird im allgemeinen bei Temperaturen zwischen etwa 0 und 130°C vorgenommen. Für die Cyclisierung in Gegenwart eines inerten organischen Lösungsmittel werden je nach Art des Lösungsmittels Temperaturen zwischen etwa 0 und 90°C bevorzugt. In den meisten Fällen kann die Cyclisierung der Säurechloride in Gegenwart von inerten organischen Lösungsmitteln aber schon bei Temperaturen zwischen etwa 0 und 40°C vorgenommen werden. Für die Cyclisierung in der Schmelze werden Temperaturen zwischen etwa 70 und 120°C bevorzugt.

Nach Beendigung der Umsetzung können die Verbindungen der Formel I auf übliche Weise isoliert werden. Durch Friedel-Crafts-Cyclisierung der Säurechloride von Zwischenprodukten der Formel IV erhaltene Verbindungen der Formel I können z.B. durch Eingiessen in ein Wasser/Eis-Gemisch oder durch Zugabe von verdünnter Mineralsäure, wie Salzsäure, Filtrieren und Waschen mit Wasser isoliert werden.

Die nach dem erfindungsgemässen Verfahren erhaltenen Verbindungen der Formel I enthalten im allgemeinen nur geringfügige Verunreinigungen. Gewünschtenfalls können sie durch Umkristallisation aus geeigneten Lösungsmitteln, wie Essigsäure, Essigsäureäthylester, Cellosolve, Äthylenglykoldimethyl- oder -diäthyläther, Aceton, Methanol oder Äthanol, oder durch Aufschlämmen in einem dieser Lösungsmittel und Abfiltrieren in analysenreine Form gebracht werden.

Die Verbindungen der Formel I fallen in Form von roten bis dunkelvioletten Kristallen an und sind zum Teil bekannt. Nach dem erfindungsgemässen Verfahren lassen sich aber auch symmetrische Thioindigoverbindungen der Formel I mit bisher nicht bekannten Substituentenkombinationen herstellen. Die Verbindungen der Formel I können, wie eingangs erwähnt, unter anderem auf an sich bekannte Weise als Küpen-, Dispersions-, Spinnmasse- und Pigmentfarbstoffe eingesetzt werden [vgl. z.B. DE-OS 2401981, 2520196, US-PS 2882277 und 3793341].

Beispiele 1–15

Herstellung von Verbindungen der Formel IV

25,5 g (0,1 Mol) 2-Chlorcarboxymethylen-5-methyl-[2H]-benzothiophen-3-on [hergestellt gemäss DE-OS 2804842] werden in 30 ml wasserfreier Essigsäure bei 20°C aufgeschlämmt und mit 11,0 g (0,1 Mol) Thiophenol versetzt. Dann wird 1½ Stunden auf 50°C erwärmt. Nach dem Abkühlen filtriert man den orangeroten Niederschlag ab, wäscht ihn mit wasserfreier Essigsäure nach und trocknet ihn bei 60°C und 13000 Pa. Man erhält 28,0 g (85,4% d.Th.) 2-Thiophenylcarboxymethylen-5-methylbenzothiophen-3-on in Form orangeroter Kristalle; Smp. 164–167°C (Zersetzung).

IR-Spektrum (KBr; w = schwach, m = mittel, s = stark):

$3115^{m}$, $1750^{s}$, $1670^{s}$, $1610^{m}$, $1545^{s}$, $1470^{s}$, $1285^{s}$, $1220^{s}$, $1165^{m}$, $1100^{s}$, $1020^{m}$, $1005^{m}$, $826^{m}$, $775^{s}$, $745^{s}$.

$R_f$ (Kieselgel/Laufmittel (v:v) Toluol:Essigsäure 70:30): 0,51.

Elementaranalyse für $C_{17}H_{12}S_2O_3$:

| | | | |
|---|---|---|---|
| Ber.: | C 62,18% | H 3,69% | S 19,53% |
| Gef.: | C 62,2 % | H 3,7 % | S 19,5 % |

In der folgenden Tabelle I sind weitere Verbindungen der Formel IV angeführt, die nach dem oben beschriebenen Verfahren hergestellt wurden.

Tabelle I

| Bsp. Nr. | Verbindung (II) | | | | Verbindung (III) | | | | $R_f$ [Kieselgel/Laufmittel (v:v) Toluol:Eisessig 70:30] |
|---|---|---|---|---|---|---|---|---|---|
| | 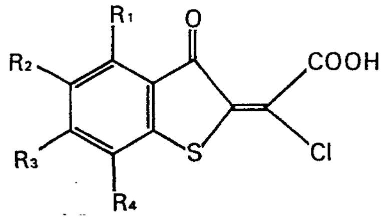 | | | | 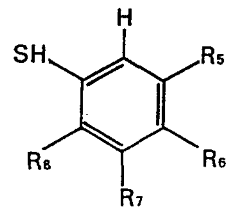 | | | | |
| | $R_1$ | $R_2$ | $R_3$ | $R_4$ | $R_5$ | $R_6$ | $R_7$ | $R_8$ | |
| 2 | H | Cl | H | H | H | Br | H | H | 0,63 |
| 3 | H | Cl | H | H | Cl | H | H | Cl | 0,52 |
| 4 | H | Br | $CH_3$ | H | Cl | H | H | Cl | 0,56 |
| 5 | H | Cl | H | H | H | $-NHCOCH_3$ | H | H | 0,17 |
| 6 | H | H | $SCH_3$ | H | H | Cl | H | H | 0,55 |
| 7 | H | Cl | H | H | H | Br | $CH_3$ | H | 0,53 |
| 8 | $CH_3$ | H | H | $CH_3$ | Cl | H | H | Cl | 0,44 |
| 9 | H | $CH_3$ | H | H | H | Br | $CH_3$ | H | 0,58 |
| 10 | H | $-CH_2CH_2CH_2$ | - | H | H | Cl | H | H | 0,61 |
| 11 | H | $n-C_4H_9$ | H | H | H | Cl | H | H | 0,58 |
| 12 | $CH_3$ | H | H | $CH_3$ | H | H | H | H | 0,59 |
| 13 | H | Br | $CH_3$ | H | Cl | H | H | Cl | 0,48 |
| 14 | H | $CH_3$ | H | H | H | $-(CH_2)_2OCOCH_3$ | H | H | 0,48 |
| 15 | H | $n-C_4H_9$ | H | H | Cl | H | H | Cl | 0,56 |

Beispiele 16-30

Herstellung von Verbindungen der Formel I aus Verbindungen der Formel IV gemäss Beispielen 1-15

11,5 g (0,035 Mol) 2-Thiophenylcarboxymethylen-5-methylbenzothiophen-3-on werden in 100 ml 1,2-Dichloräthan aufgeschlämmt, mit 12,6 g (0,105 Mol) Thionylchlorid und 2 ml N,N-Dimethylformamid versetzt und 4 Stunden auf 60°C erwärmt. Dann wird die erhaltene dunkelrote Lösung eingedampft, und das ausgefallene orange Pulver wird zu 85 g (0,36 Mol) Aluminiumtrichlorid in 85 ml 1,2-Dichloräthan gegeben, wobei mit einem externen Eisbad die Temperatur auf 20°C gehalten wird. Nach 15stündigem Rühren bei 20-25°C wird die schwarze Suspension auf Eis gegossen, 20 Minuten gerührt, und die entstandenen roten Kristalle werden nach Zugabe von 50 ml Essigsäureäthylester abfiltriert. Diese werden mit 25 ml Essigsäureäthylester gewaschen und bei 60°C und 13000 Pa getrocknet. Es fallen 10,4 g (96% d.Th.) 5-Methylthioindigo in Form roter Kristalle an; Smp. > 250°C.

IR-Spektrum (KBr; w = schwach, m = mittel, s = stark):

$1655^s$, $1600^m$, $1475^m$, $1385^s$, $1225^m$, $1175^m$, $1080^s$, $1060^w$, $900^s$, $815^m$, $775^m$, $745^s$.

Elementaranalyse für $C_{17}H_{10}O_2S_2$:

Ber.: C 65,79% H 3,25% S 20,66% O 10,31%
Gef.: C 65,65% H 3,26% S 20,13% O 10,62%

$R_f$ [Kieselgel/Laufmittel Toluol:Essigsäure (v:v) = 70:30]: 0,78.

In der folgenden Tabelle II sind weitere Verbindungen der Formel I angeführt, die nach dem oben beschriebenen Verfahren aus den entsprechenden Verbindungen der Formel IV hergestellt wurden.

Tabelle II

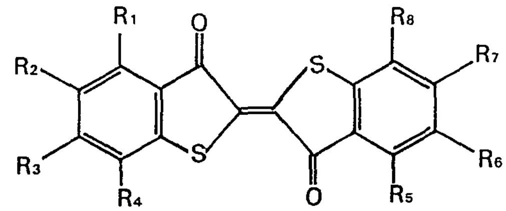

| Bsp. Nr. | Ausgangs-material gemäss | $R_1$ | $R_2$ | $R_3$ | $R_4$ | $R_5$ | $R_6$ | $R_7$ | $R_8$ | $R_f$ [Kieselgel/ Laufmittel (v:v) Toluol:Essigsäure 70:30] | Farbe/ Substrat |
|---|---|---|---|---|---|---|---|---|---|---|---|
| 17 | Bsp. 2 | H | Cl | H | H | H | Br | H | H | schwer löslich | violett-rosa/PES |
| 18 | Bsp. 3 | H | Cl | H | H | Cl | H | H | Cl | 0,88 | blau-rot/PVC |
| 19 | Bsp. 4 | H | Br | $CH_3$ | H | Cl | H | H | Cl | 0,88 | blau-rot/PVC |
| 20 | Bsp. 5 | H | Cl | H | H | H | $-NHCOCH_3$ | H | H | 0,44 | blauvio-lett/PES |
| 21 | Bsp. 6 | H | H | $SCH_3$ | H | H | Cl | H | H | 0,89 | vio-lett/PES |
| 22 | Bsp. 7 | H | Cl | H | H | H | Br | $CH_3$ | H | 0,88 | vio-lett/PVC |
| 23 | Bsp. 8 | $CH_3$ | H | H | $CH_3$ | Cl | H | H | Cl | 0,86 | violett-rosa/PES |
| 24 | Bsp. 9 | H | $CH_3$ | H | H | H | Br | $CH_3$ | H | 0,88 | vio-lett/PES |
| 25 | Bsp. 10 | H | $-CH_2-CH_2-CH_2-$ | | H | H | Cl | H | H | 0,85 | violett-rosa/PES |
| 26 | Bsp. 11 | H | $n-C_4H_9$ | H | H | H | Cl | H | H | 0,90 | violett-rosa/PES |
| 27 | Bsp. 12 | $CH_3$ | H | H | $CH_3$ | H | H | H | H | 0,83 | rot/PES |
| 28 | Bsp. 13 | H | Br | $CH_3$ | H | Cl | H | H | Cl | 0,89 | rotbraun/PES |
| 29 | Bsp. 14 | H | $CH_3$ | H | H | H | $-(CH_2)_2OCOCH_3$ | H | H | 0,72 | rot/PES |
| 30 | Bsp. 15 | H | $n-C_4-H_9$ | H | H | Cl | H | H | Cl | 0,91 | violett-rosa/PES |

PES = Polyesterfaser, gefärbt gemäss Verwendungsbeispiel A
PVC = Polyvinylchlorid, gefärbt gemäss Verwendungsbeispiel B

Verwendungsbeispiele

Beispiel A

Ein zur Faserherstellung geeignetes unmattiertes Polyäthylenterephthalat-Granulat wird mit 1 Gew.-% des gemäss Beispiel 16 hergestellten 5-Methylthioindigo auf einer Schüttelmaschine 15 Minuten geschüttelt. Hierauf werden die Granulatkörper auf einer Schmelzspinnanlage (285°C ±3°C, Verweilzeit in der Spinnmaschine ca. 5 Minuten) zu Fäden versponnen, die auf einer Streckzwirnanlage verstreckt und aufgespult werden. Man erhält eine brillante, bordeauxrote Färbung, die sich vor allem durch eine hohe Lichtechtheit auszeichnet.

Beispiel B

65 Gew.-Teile stabilisiertes Polyvinylchlorid, 35 Gew.-Teile Dioctylphthalat und 0,2 Gew.-Teile des gemäss Beispiel 18 erhaltenen Pigmentfarbstoffes werden miteinander verrührt und dann auf einem Zweiwalzenkalander während 7 Minuten bei 140°C hin- und hergewalzt. Man erhält eine blaurote Färbung von guter Licht- und Migrationsechtheit.

Beispiel C

100 Gew.-Teile Polyäthylenterephthalatgranulat und 0,2 Gew.-Teile des nach Beispiel 17 hergestellten Pigmentfarbstoffes werden in einer

Mischtrommel intensiv vermischt und danach während ca. 24 Stunden bei 100°C am Vakuum bei $135 \times 10^{-3}$ Pa getrocknet. Das so behandelte Granulat wird bei ca. 280°C in einem Einwellenextruder zu einem Band extrudiert. Man erhält ein stark violett gefärbtes Band mit ausgezeichneten Lichtechtheiten.

**Patentansprüche**

1. Ein Verfahren zur Herstellung einer Verbindung der Formel I

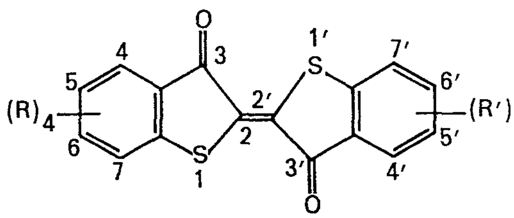
(I),

worin
zwei R bzw. R′ Wasserstoff oder Alkyl mit 1–12 C-Atomen,
ein R bzw. R′ Wasserstoff, ein Halogenatom oder Alkyl mit 1–12 C-Atomen und
ein R bzw. R′ Wasserstoff, ein Halogenatom, Alkyl, Alkoxy, Alkylthio, Alkoxyalkyl, Alkanoyloxyalkyl oder Alkylcarbamoyl mit je 1–12 C-Atomen in den Alkyl- und Alkoxyteilen und 2–13 C-Atomen in den Alkanoylteilen oder zwei benachbarte Substituenten R bzw. R′ zusammen Trimethylen oder Tetramethylen und die restlichen R Wasserstoff darstellen, mit der Massgabe, dass mindestens ein R ungleich einem Substituenten R′ ist, dadurch gekennzeichnet, dass man eine Verbindung der Formel II

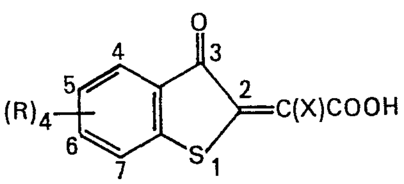
(II)

mit einer Verbindung der Formel III

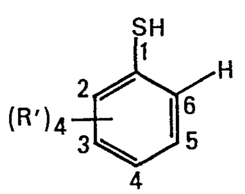
(III)

zu einer Verbindung der Formel IV

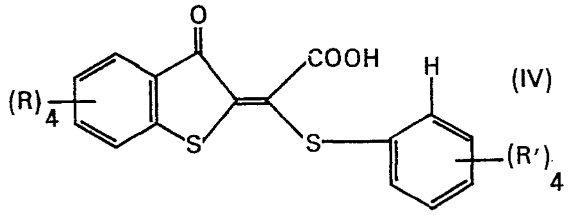
(IV)

umsetzt, die Verbindung der Formel IV entweder direkt durch Wasserabspaltung zu einer Verbindung der Formel I cyclisiert oder die Verbindung der Formel IV in das entsprechende Säurechlorid überführt und das Säurechlorid in Gegenwart eines Friedel-Crafts-Katalysators zu einer Verbindung der Formel I cyclisiert, wobei X Chlor, Brom oder Fluor bedeutet und für R bzw. R′ das unter Formel I Angegebene gilt.

2. Ein Verfahren nach Anspruch 1, dadurch gekennzeichnet, dass man eine Verbindung der Formel II und/oder III verwendet, worin zwei R bzw. R′ Wasserstoff oder Alkyl mit 1–4 C-Atomen, ein R bzw. R′ Wasserstoff, Chlor, Brom oder Alkyl mit 1–4 C-Atomen und ein R bzw. R′ Wasserstoff, Chlor, Brom, Alkyl, Alkoxy, Alkylthio, $-(CH_2)_2-O-$Alkyl,

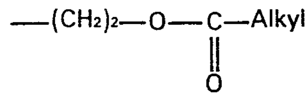

oder -NHCO-Alkyl mit je 1–4 C-Atomen in den Alkylteilen und X Chlor bedeuten.

3. Ein Verfahren nach Anspruch 1, dadurch gekennzeichnet, dass man eine Verbindung der Formel II und/oder III verwendet, worin zwei R bzw. R′ Wasserstoff bedeuten und die restlichen R bzw. R′ ungleich Wasserstoff sind, mit der Massgabe, dass die R bzw. R′ in 4- und 5-Stellung nicht gleichzeitig ungleich Wasserstoff sind.

4. Ein Verfahren nach Anspruch 1, dadurch gekennzeichnet, dass man eine Verbindung der Formel II und/oder III verwendet, worin entweder die R in 5- und 6-Stellung und die R′ in 3- und 4-Stellung Wasserstoff und die restlichen R bzw. R′ ungleich Wasserstoff sind, oder worin die R in 4- und 7-Stellung bzw. die R′ in 2- und 5-Stellung Wasserstoff und die restlichen R bzw. R′ ungleich Wasserstoff sind.

5. Ein Verfahren nach Anspruch 1, dadurch gekennzeichnet, dass man eine Verbindung der Formel II oder III verwendet, worin das R in 5-, 6- oder 7-Stellung bzw. das R′ in 2-, 3- oder 4-Stellung ungleich Wasserstoff ist.

6. Ein Verfahren nach Anspruch 1, dadurch gekennzeichnet, dass man eine Verbindung der Formel II und III verwendet, worin X Chlor, die R in 4- und 7-Stellung Wasserstoff, das R in 5-Stellung Wasserstoff, Chlor, Brom oder Alkyl mit 1–4 C-Atomen, das R in 6-Stellung Wasserstoff, Alkyl oder Alkylthio mit je 1–4 C-Atomen, oder die R in 5- und 6-Stellung zusammen Trimethylen und die restlichen R Wasserstoff, die R′ in 2- und 5-Stellung Wasserstoff, das R′ in 3-Stellung Wasserstoff oder Alkyl mit 1–4 C- Atomen und das R′ in 4-Stellung Chlor, Brom, -NHCO-Alkyl oder $-(CH_2)_2OCO$-Alkyl mit je 1–4 C-Atomen in den Alkylteilen oder die R′ in 2- und 5-Stellung Chlor oder Brom und die R′ in 3- und 4-Stellung Wasserstoff bedeuten.

7. Ein Verfahren nach Anspruch 1, dadurch gekennzeichnet, dass man eine Verbindung der Formel II und III verwendet, worin X Chlor, die R in 4- und 7-Stellung Alkyl mit 1–4 C-Atomen, die R′ in 2- und 5-Stellung Wasserstoff, Chlor oder Brom und die restlichen R und R′ Wasserstoff bedeuten.

8. Ein Verfahren nach Anspruch 1, dadurch gekennzeichnet, dass man die Umsetzung einer Verbindung der Formel II mit einer Verbindung der Formel III in Gegenwart eines inerten organischen Lösungsmittels bei einer Temperatur zwischen etwa 0 und 120°C vornimmt.

9. Ein Verfahren nach Anspruch 1, dadurch gekennzeichnet, dass man die Cyclisierung des Säurechlorids einer Verbindung der Formel IV in Gegenwart eines inerten organischen Lösungsmittels bei einer Temperatur zwischen etwa 0 und 90°C oder in der Schmelze unter Zusatz von Natrium- und/oder Kaliumchlorid bei einer Temperatur zwischen etwa 70 und 120°C vornimmt.

10. Ein Verfahren nach Anspruch 1, dadurch gekennzeichnet, dass man für die Cyclisierung des Säurechlorids einer Verbindung der Formel IV Aluminiumtrichlorid als Friedel-Crafts-Katalysator verwendet.

11. Eine Verbindung der Formel IV

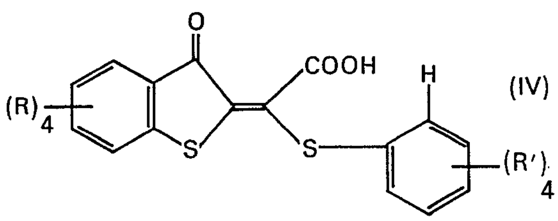

(IV)

worin
zwei R bzw. R′ Wasserstoff oder Alkyl mit 1–12 C-Atomen,
ein R bzw. R′ Wasserstoff, ein Halogenatom oder Alkyl mit 1-12 C-Atomen und
ein R bzw. R′ Wasserstoff, ein Halogenatom, Alkyl, Alkoxy, Alkylthio, Alkoxyalkyl, Alkanoyloxyalkyl oder Alkylcarbamoyl mit je 1-12 C-Atomen in den Alkyl- und Alkoxyteilen und 2–13 C-Atomen in den Alkanoylteilen oder zwei benachbarte Substituenten R bzw. R′ zusammen Trimethylen oder Tetramethylen und die restlichen R Wasserstoff darstellen,
mit der Massgabe, dass mindestens eines von R ungleich einem Substituenten R′ ist.

**Claims**

1. A process for the production of a compound of the formula I

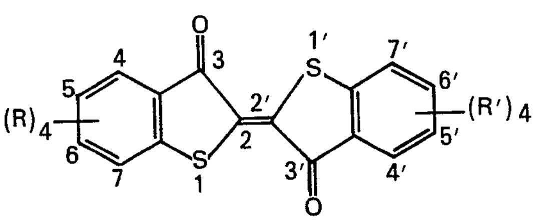

(I),

wherein two substituents R or R′ are hydrogen or alkyl of 1 to 12 carbon atoms, one R or R′ is hydrogen, a halogen atom or alkyl of 1 to 12 carbon atoms, and one R or R′ is hydrogen, a halogen atom, alkyl, alkoxy, alkylthio, alkoxyalkyl, alkanoyloxyalkyl or alkylcarbamoyl, each containing 1 to 12 carbon atoms in the alkyl and alkoxy moieties and 2 to 13 carbon atoms in the alkanoyl moieties, or two adjacent substituents R or R′ together are trimethylene or tetramethylene and the other substituents R are hydrogen, with the proviso that at least one substituent R is different from a substituent R′, which process comprises reacting a compound of the formula II

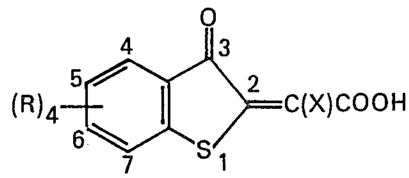

(II)

with a compound of the formula III

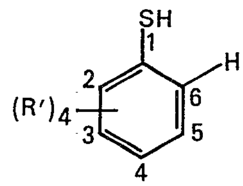

(III)

to give a compound of the formula IV

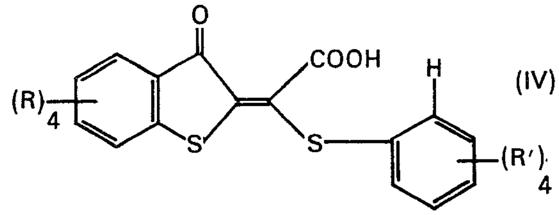

(IV)

cyclising said compound of the formula IV either direct by splitting off water to give a compound of the formula I, or converting said compound of the formula IV into the corresponding acid chloride and cyclising this latter, in the presence of a Friedel-Crafts catalyst, to give a compound of the formula I, in which formulae II, III and IV above X is chlorine, bromine or fluorine and R and R′ are as defined for formula I.

2. A process according to claim 1 which comprises the use of a compound of the formula II and/or III, wherein two substituents R or R′ are hydrogen or alkyl of 1 to 4 carbon atoms, one R or R′ is hydrogen, chlorine, bromine or alkyl of 1 to 4 carbon atoms, and one R or R′ is hydrogen, chlorine, bromine, alkyl, alkoxy, alkylthio, $-(CH_2)_2-O-$alkyl,

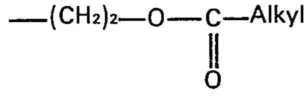

or -NHCO-alkyl, each containing 1 to 4 carbon atoms in the alkyl moieties, and X is chlorine.

3. A process according to claim 1 which comprises the use of a compound of the formula II and/or III, wherein two substituents R or R′ are hydrogen and the others are different from hydrogen, with the proviso that the substituents R and R′ in the 4- and 5-position are not simultaneously different from hydrogen.

4. A process according to claim 1 which comprises the use of a compound of the formula II and/or III, wherein either the substituents R in the 5- and 6-position and the substituents R′ in the 3- and 4-position are hydrogen and the other substituents R and R′ are different from hydrogen, or wherein the substituents R in the 4- and 7-position and the substituents R′ in the 2- and 5-position are hydrogen and the other substituents R and R′ are different from hydrogen.

5. A process according to claim 1 which comprises the use of a compound of the formula II or III, wherein the substituent R in the 5-, 6- or 7-position or the substituent R′ in the 2-, 3- or 4-position is different from hydrogen.

6. A process according to claim 1 which comprises the use of a compound of the formula II and III, wherein X is chlorine, the substituents R in the 4- and 7-position are hydrogen, the substituent R in the 5-position is hydrogen, chlorine, bromine or alkyl of 1 to 4 carbon atoms, the substituent R in the 6-position is hydrogen, alkyl or alkylthio, each containing 1 to 4 carbon atoms, or the substituents R in the 5- and 6-position together are trimethylene and the others are hydrogen, the substituents R′ in the 2- and 5-position are hydrogen, the substituent R′ in the 3-position is hydrogen or alkyl of 1 to 4 carbon atoms, and the substituent R′ in the 4-position is chlorine, bromine, -NHCO-alkyl or $-(CH_2)_2$-OCO-alkyl, each containing 1 to 4 carbon atoms in the alkyl moieties, or the substituents R′ in the 2- and 5-position are chlorine or bromine and the substituents R′ in the 3- and 4-position are hydrogen.

7. A process according to claim 1 which comprises the use of a compound of the formula II and III, wherein X is chlorine, the substituents R in the 4- and 7-position are alkyl of 1 to 4 carbon atoms, the substituents R′ in the 2- and 5-position are hydrogen, chlorine or bromine, and the other substituents R and R′ are hydrogen.

8. A process according to claim 1, wherein the reaction of a compound of the formula II with a compound of the formula III is carried out in the presence of an inert organic solvent in the temperature range from about 0° to 120°C.

9. A process according to claim 1, wherein the cyclisation of the acid chloride of a compound of the formula IV is carried out in the presence of an inert organic solvent in the temperature range from about 0° to 90°C, or in the melt, with the addition of sodium and/or potassium chloride, in the temperature range from about 70° to 120°C.

10. A process according to claim 1, wherein aluminium trichloride is used as Friedel-Crafts catalyst for the cyclisation of the acid chloride of a compound of the formula IV.

11. A compound of the formula IV

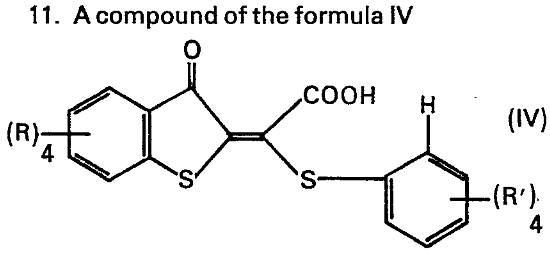

(IV)

wherein two substituents R or R′ are hydrogen or alkyl of 1 to 12 carbon atoms, one R or R′ is hydrogen, a halogen atom or alkyl of 1 to 12 carbon atoms, and one R or R′ is hydrogen, a halogen atom, alkyl, alkoxy, alkylthio, alkoxyalkyl, alkanoyloxyalkyl or alkylcarbamoyl, each containing 1 to 12 carbon atoms in the alkyl and alkoxy moieties and 2 to 13 carbon atoms in the alkanoyl moieties, or two adjacent substituents R or R′ together are trimethylene or tetramethylene and the other substituents R are hydrogen, with the proviso that at least one substituent R is different from a substituent R′.

## Revendications

1. Procédé de préparation d'un composé de formule I:

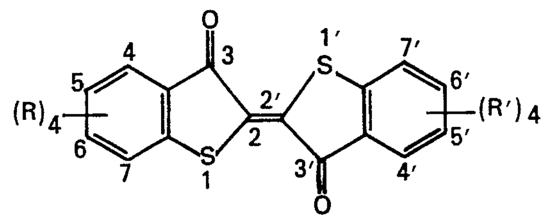

(I),

dans laquelle:
deux R ou R′ représentent l'hydrogène ou des restes alkyle ayant 1 à 12 atomes de carbone,
un R ou R′ représente l'hydrogène, un atome d'halogène ou un reste alkyle ayant 1 à 12 atomes de carbone, et
un R ou R′ représente l'hydrogène, un atome d'halogène, un reste alkyle, alcoxy, alkylthio, alcoxyalkyle, alcanoyloxyalkyle ou alkylcarbamoyle ayant à chaque fois 1 à 12 atomes de carbone dans les parties alkyle et alcoxy et 2 à 13 atomes de carbone dans les parties alcanoyle, ou bien deux substituants R ou R′ voisins représentent ensemble le reste triméthylène ou tétraméthylène et les autres R représentent l'hydrogène, à la condition qu'au moins un R soit différent d'un substituant R′, caractérisé par le fait que l'on fait réagir un composé de formule II:

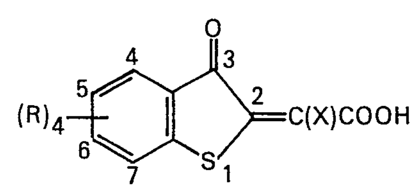

(II)

avec un composé de formule III:

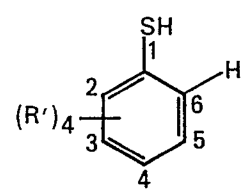

(III)

pour avoir un composé de formule IV:

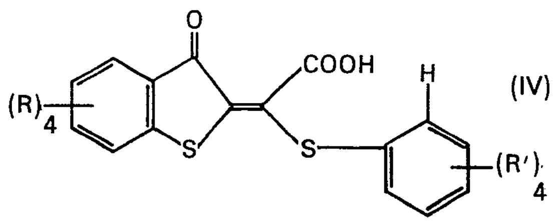

(IV)

et, soit que l'on cyclise le composé de formule IV directement par déshydratation pour avoir un composé de formule I, soit que l'on transforme le composé de formule IV en le chlorure d'acide correspondant et que l'on cyclise le chlorure d'acide en présence d'un catalyseur de Friedel-Crafts pour avoir un composé de formule I, X représentant le chlore, le brome ou le fluor, et ce qui a été indiqué sous la formule I pour R ou R′ étant valable.

2. Procédé selon la revendication 1, caractérisé par le fait que l'on utilise un composé des formules II et/ou III, dans lequel deux R ou R′ représentent l'hydrogène ou des restes alkyle ayant 1 à 4 atomes de carbone, un R ou R′ représente l'hydrogène, le chlore, le brome ou un reste alkyle ayant 1 à 4 atomes de carbone et un R ou R′ représente l'hydrogène, le chlore, le brome, un reste alkyle, alcoxy, alkylthio, $-(CH_2)_2-O-$alkyle,

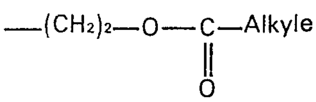

ou -NHCO-alkyle ayant à chaque fois 1 à 4 atomes de carbone dans les parties alkyle et X représente le chlore.

3. Procédé selon la revendication 1, caractérisé par le fait que l'on utilise un composé des formules II et/ou III, dans lequel deux R ou R′ représentent l'hydrogène, et les autres R ou R′ sont différents de l'hydrogène, à la condition que les R ou R′ sur les positions 4 et 5 ne soient pas en même temps différents de l'hydrogène.

4. Procédé selon la revendication 1, caractérisé par le fait que l'on utilise un composé des formules II et/ou III, dans lequel, soit les R sur les positions 5 et 6 et les R′ sur les positions 3 et 4 sont l'hydrogène et les autres R ou R′ sont différents de l'hydrogène, soit les R sur les positions 4 et 7 ou les R′ sur les positions 2 et 5 sont l'hydrogène et les autres R ou R′ sont différents de l'hydrogène.

5. Procédé selon la revendication 1, caractérisé par le fait que l'on utilise un composé des formules II ou III, dans lequel le R sur les positions 5, 6 ou 7 ou le R′ sur les positions 2, 3 ou 4 est différent de l'hydrogène.

6. Procédé selon la revendication 1, caractérisé par le fait que l'on utilise un composé des formules II et III, dans lequel X représente le chlore, les R sur les positions 4 et 7 représentent l'hydrogène, le R sur la position 5 représente l'hydrogène, le chlore, le brome ou un reste alkyle ayant 1 à 4 atomes de carbone, le R sur la position 6 représente l'hydrogène, un reste alkyle ou alkylthio ayant à chaque fois 1 à 4 atomes de carbone, ou bien les R sur les positions 5 et 6 représentent ensemble le reste triméthylène, et les autres R représentent l'hydrogène, les R′ sur les positions 2 et 5 représentent l'hydrogène, le R′ sur la position 3 représente l'hydrogène ou un reste alkyle ayant 1 à 4 atomes de carbone, et le R′ sur la position 4 représente le chlore, le brome, -NHCO-alkyle ou $-(CH_2)_2-OCO-$alkyle ayant à chaque fois 1 à 4 atomes de carbone dans les parties alkyle ou bien les R′ sur les positions 2 et 5 représentent le chlore ou le brome et les R′ sur les positions 3 et 4 représentent l'hydrogène.

7. Procédé selon la revendication 1, caractérisé par le fait que l'on utilise un composé des formules II et III, dans lequel X représente le chlore, les R sur les positions 4 et 7 représentent des restes alkyle ayant 1 à 4 atomes de carbone, les R′ sur les positions 2 et 5 représentent l'hydrogène, le chlore ou le brome et les autres R et R′ représentent l'hydrogène.

8. Procédé selon la revendication 1, caractérisé par le fait que l'on effectue la réaction d'un composé de formule II avec un composé de formule III, en présence d'un solvant organique inerte, à une température comprise entre environ 0 et 120°C.

9. Procédé selon la revendication 1, caractérisé par le fait que l'on effectue la cyclisation du chlorure d'acide d'un composé de formule IV, en présence d'un solvant organique inerte, à une température comprise entre environ 0 et 90°C, ou bien dans la masse fondue avec addition de chlorure de sodium et/ou de potassium à une température comprise entre environ 70 et 120°C.

10. Procédé selon la revendication 1, caractérisé par le fait que l'on utilise pour la cyclisation du chlorure d'acide d'un composé de formule IV, du trichlorure d'aluminium comme catalyseur de Friedel-Crafts.

11. Composé de formule IV:

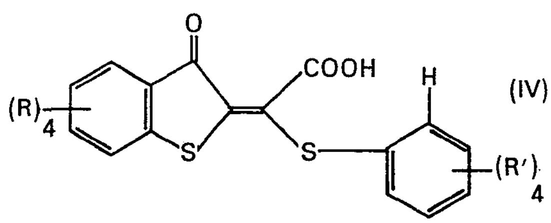

(IV)

dans laquelle:
deux R ou R′ représentent l'hydrogène ou des restes alkyle ayant 1 à 12 atomes de carbone,
un R ou R′ représente l'hydrogène, un atome d'halogène ou un reste alkyle ayant 1 à 12 atomes de carbone, et
un R ou R′ représente l'hydrogène, un atome d'halogène, un reste alkyle, alcoxy, alkylthio, alcoxyalkyle, alcanoyloxyalkyle ou alkylcarbamoyle ayant à chaque fois 1 à 12 atomes de carbone dans les parties alkyle et alcoxy, et 2 à 13 atomes de carbone dans les parties alcanoyle ou bien deux substituants R ou R′ voisins représentant ensemble le reste triméthylène ou tétraméthylène, et les autres R représentent l'hydrogène, à la condition qu'au moins un des R soit différent d'un substituant R′.